# EUROPEAN PATENT SPECIFICATION

(11) **EP 3 864 154 B1**
(45) Date of publication and mention of the grant of the patent: **25.12.2024**
(21) Application number: 19794277.4
(22) Date of filing: 09.10.2019
(51) Int. Cl.: C12N 15/63, C12N 15/67

(54) **NUCLEIC ACID TO ACTIVATE GENE EXPRESSION AND PROTEIN PRODUCTION**
NUKLEINSÄURE ZUR AKTIVIERUNG DER GENEXPRESSION UND DER PROTEINPRODUKTION
ACIDE NUCLÉIQUE POUR ACTIVER L'EXPRESSION GÉNIQUE ET LA PRODUCTION DE PROTÉINES

(30) Priority: 09.10.2018 PT 2018115073
(43) Date of publication of application: 18.08.2021
(73) Proprietor: Instituto De Biologia Molecular E Celular - IBMC, 4200-135 Porto (PT)
(72) Inventor: OLIVEIRA, Marta, 4200-135 Porto (PT); JESUS, Ana, 4200-135 Porto (PT); FREITAS, Jaime, 4200-135 Porto (PT); MOREIRA, Alexandra, 4200-135 Porto (PT); RIBEIRO BESSA, José Carlos, 4200-135 Porto (PT)
(74) Representative: ABG Intellectual Property Law, S.L.
(86) International application number: PCT/PT2019/050035
(87) International publication number: WO 2020/076174

(56) References cited:
- WO-A2-01/71042
- US-A1- 2005 208 558
- US-A1- 2008 241 883
- LLAMAZARES S ET AL: "POLO ENCODES A PROTEIN KINASE HOMOLOG REQUIRED FOR MITOSIS IN DROSOPHILA", GENES AND DEVELOPMENT, COLD SPRING HARBOR LABORATORY PRESS, PLAINVIEW, NY, US, vol. 5, no. 12A, 1 January 1991 (1991-01-01), pages 2153 - 2165, XP001024889, ISSN: 0890-9369
- DATABASE EMBL [online] 4 December 1991 (1991-12-04), "D.melanogaster poLo mRNA", retrieved from EBI accession no. EM_STD:X63361 Database accession no. X63361
- TECHNICAL BULLETIN: "SP6 RNA Polymerase is a DNA-dependent RNA", 21 May 2002 (2002-05-21), pages 1 - 7, XP055650774, Retrieved from the Internet <URL:https://tools.thermofisher.com/content/sfs/brochures/711-01110%20TB-SP6%20RNA%20Polyme.pdf> [retrieved on 20191209]
- STEPHEN T THIBAULT ET AL: "A complementary transposon tool kit for Drosophila melanogaster using P and piggyBac", NATURE GENETICS., vol. 36, no. 3, 1 March 2004 (2004-03-01), NEW YORK, US, pages 283 - 287, XP055650636, ISSN: 1061-4036, DOI: 10.1038/ng1314
- DATABASE EMBL [online] 30 April 2005 (2005-04-30), "e01048-5prime Exelixis piggyBac RB insertions Drosophila melanogaster genomic Sequence recovered from 5' end of piggyBac, genomic survey sequence.", retrieved from EBI accession no. EM_GSS:CZ478874 Database accession no. CZ478874
- X. HONG ET AL: "Microarray-Based Capture of Novel Expressed Cell Type-Specific Transfrags (CoNECT) to Annotate Tissue-Specific Transcription in Drosophila melanogaster", G3: GENES|GENOMES|GENETICS, vol. 2, no. 8, 1 August 2012 (2012-08-01), pages 873 - 882, XP055650638, DOI: 10.1534/g3.112.003194
- DATABASE EMBL [online] 11 May 2012 (2012-05-11), "TSA: Drosophila melanogaster isotig05270.Dmov mRNA sequence.", retrieved from EBI accession no. EM_TSA:JV213422 Database accession no. JV213422
- INA ANREITER ET AL: "Epigenetic mechanisms modulate differences in Drosophila foraging behavior", PROCEEDINGS OF THE NATIONAL ACADEMY OF SCIENCES, vol. 114, no. 47, 16 October 2017 (2017-10-16), pages 12518 - 12523, XP055650642, ISSN: 0027-8424, DOI: 10.1073/pnas.1710770114
- DATABASE EMBL [online] 29 September 2017 (2017-09-29), "Drosophila melanogaster strain sitter (fors) chromosome 3L.", retrieved from EBI accession no. EM_STD:CP023331 Database accession no. CP023331

## Description

### Technical field of the invention

The present invention refers to a method for increasing the efficiency of recombinant protein production by employing a composition that comprises a nucleic acid comprising at least one copy of SEQ ID nr. 1 which is used to incorporate in the sequence of a genetic construct or a gene expression vector, in zebrafish, fruit fly or other model organisms, as well as mammalian or human cells, to enhance protein production and be advantageously used as a new tool to boost protein yield in biotechnology and/or biopharmaceutical applications. Thus, the present invention falls within the technical field of chemistry, biochemistry, molecular biology, microorganism's genetic engineering, recombinant-DNA technology.

### State of the art

The efficiency of recombinant protein production is an important bottleneck in most biopharmaceuticals and biotechnology industries and strategies to increase productivity usually rely on improvement in host cell growth media composition (as disclosed in documents US2017218328 and WO9429435) or on controlling the cell growth process, through induction or enhancement (as described in documents US5089397 and US2018135008, respectively); or through a stepwise process for scaling-up the culture. Another strategy to tackle this problem regards the use of more efficient expression vectors, trough addition of several types of expression enhancer elements, as described for example in documents CN107090441, CN107653266, KR20170140925 and CN104975018. The expression vectors are genetically engineered constructs for the introduction of genes and expression of proteins of interest in the host cells. Expression vectors may contain different types of promoters (as disclosed in US2019055580), as well as other regulatory elements, upstream and/or downstream of the gene coding sequence, so that more efficient expression of the gene of interest occurs. For example, document US2008241883 discloses enhancer elements for recombinant expression vectors to promote expression of a recombinant protein in a host cell. However, these elements are very long in length, with 2329 and 2422 base pairs, which makes them difficult to sub-clone and brings the drawback of adding considerable size to the backbone expression vector, making it more difficult to amplify, manipulate and transfect. Certain enhancers are functional only in specific host cells, as exemplified by the enhancer described in documents CN107653266 or CN105238816, which are designed only for insect cells, or the vector disclosed in US20110195451 which is designed only for animal cells. Other enhancer sequences are specific for given genes, as exemplified in documents US2010151520 and US2003013867. Thus, despite advances in the understanding of various sequences and other factors that can affect expression of recombinant proteins, needs remain to improve the yields of recombinant proteins, particularly of recombinant proteins that are intended for therapeutic or other specialized biotechnology applications. Desirably, this could be solved by small enhancer sequences that could be chemically synthesised and easily sub-cloned, with a "universal" effect in various types of host cells and different target genes without interfering with the gene coding region. A sequence with all these features, however, is still missing from the prior art.

These facts are disclosed in order to illustrate the technical problem addressed by the present disclosure.

### Summary of the Invention

We found and characterized a new short 28 nucleotide sequence, herein named iPLUS - increase in Protein Levels Universal Sequence - and specified in SEQID nr. 1, that strongly increases protein production *in vivo,* in several model systems including fruit fly, zebrafish and in human cell lines. This sequence can be synthetized *in vitro* and used in compositions, vectors, recombinant cell lines or kits where it dramatically increases protein production, for example of the Polo protein or of the green fluorescent protein (GFP) protein. Through specific methods, the 28 nucleotide sequence, or smaller functional fragments of this sequence, can be inserted in the sequence of a gene, of an expression vector or of a reporter vector, to enhance protein production.

As such, in a first aspect the present invention refers to a nucleic acid consisting of the 28-oligonucleotide with the base sequence of SEQ ID nr.1 or consisting of tandem repeats of the 28-oligonucleotide with the base sequence of SEQ ID nr. 1, according to claim 1.

It is disclosed a nucleic acid for increasing the expression of a protein of interest not forming part of the present invention characterized by, comprising at least one copy of a truncated sequence of the nucleotide base sequence SEQID nr.1., between 4 and 27 nucleotides, most preferably 22 nucleotides.

A second aspect of the present invention refers to a composition for increasing the expression of a protein of interest comprising:
(i) a nucleic acid as described in the first aspect of the invention or a nucleic acid consisting of the sequence SEQ ID nr.1 or consisting of tandem repeats of the sequence SEQ ID nr. 1 having restriction enzyme recognition sequences upstream, downstream or at both ends of the nucleic acid of the first aspect of the invention;
(ii) a buffer, such as Tris-HCl;
(iii) a divalent ion chelating agent, such as EDTA; and (iv) combinations thereof, according to claim 2.

The present invention also refers to a genetic construct for increasing the expression of a protein of interestcomprising at least one copy of the nucleic acid with SEQ ID nr.1, as described in the first aspect of the invention, downstream of the gene of interest coding region, wherein the genetic construct for increasing recombinant protein expression is an expression vector selected from the group consisting of a recombinant eukaryotic plasmid expression vector, a recombinant viral expression vector, a recombinant bacteriophage expression vector, a recombinant yeast mini-chromosome expression vector, a recombinant plant vector, a recombinant bacterial artificial chromosome expression vector, and a recombinant yeast expression plasmid vector, according to claim 3.

The present invention also refers to the host cell for increased expression of a protein of interest comprising at least one exogenous copy of the nucleic acid described in the first aspect of the invention, introduced downstream of the gene of interest coding region for example through gene editing, wherein the host cell is selected from the group consisting of a mammalian host cell, such as a HeLa cell, a Chinese Hamster Ovary cell, a COS cell, a Vero cell, an SP2/0 cell, an NS/0 myeloma cell, a human embryonic kidney 293 cell, a baby hamster kidney cell, a human B cell, a human T Cell Jurkat, a neuronal cell, a CV-I /EBNA cell, an L cell, a 3T3 cell, an HEPG2 cell, and an MDCK cell; a fish host cell, such as zebrafish; a plant host cell, such as Nicotania tabacum, Arabidopsis, Lactuca sativa, protoplasts and suspension cells; a fungal host cell, such as Aspergillus, Neurospora, Saccharomyces, Pichia, Hansenula, Schizosaccharomyces, Kluyveromyces, Yarrowia, and Candida cells; an algal host cell, such as Synechocystis and other Cyanobacteria cells; a nematode host cell; and a protozoan host cell; and wherein the host cell is not a human germ cell, according to claim 4.

In another aspect of the present invention, the host cell for increased expression of a protein of interest comprises, stably or transiently, the said genetic constructs or expression vectors described in claim 3 wherein the host cell is selected from the group consisting of a mammalian host cell, such asa HeLa cell, a Chinese Hamster Ovary cell, a COS cell, a Vero cell, an SP2/0 cell, an NS/0 myeloma cell, a human embryonic kidney 293 cell, a baby hamster kidney cell, a human B cell, a human T cell Jurkat, a neuronal cell, a pancreatic cell, a CV-I /EBNA cell, an L cell, a 3T3 cell, an HEPG2 cell, and an MDCK cell; a fish host cell, such aszebrafish and zebrafish cell lines; a plant host cell, such as Nicotania tabacum, Arabidopsis, Lactuca sativa, protoplasts, and suspension cells; a fungal host cell, such as Aspergillus, Neurospora, Saccharomyces, Pichia, Hansenula, Schizosaccharomyces, Kluyveromyces, Yarrowia, and Candida cells; an algal host cell, such as Synechocystis and other Cyanobacteria cells; a nematode host cell; a protozoan host cell; and a prokaryotic host cell such as Escherichia coli, *Erwinia chrysanthemi* and *Clostridium histolyticum cells;* and wherein the host cell is not a human germ cell according to claim 5.

A sixth aspect of the present invention refers to a method for increasing the expression of a protein of interest in a host cell comprising the steps of:
*a)* Synthesizing a nucleic acid for increasing the expression of a protein of interest comprising at least one copy of the 28-oligonucleotide with the base sequence of SEQ ID nr. 1 and preparing compositions comprising said nucleic acid, a buffer such as Tris_HCl, a divalent ion chelating agent, such as EDTA, and combinations thereof;
*b)* Contacting the nucleic acid composition with a linearized expression vector encoding for a protein of interest and ligating the nucleic acid downstream of the gene coding sequence, for transcription at the 3' untranslated region (3'UTR) through incubation with a DNA Ligase in the appropriate buffer and temperature conditions, so as to obtain an expression vector comprising at least one copy of the nucleic acid with SEQ ID nr. 1 downstream of the gene of interest coding region;
*c)* Introducing the said expression vectors or the said nucleic acid for increasing the expression of a protein of interest comprising at least one copy of the 28-oligonucleotide with the base sequence of SEQ ID nr. 1 into a host cell, trough transient or stable transformation, transduction, transfection or trough gene editing protocols, wherein said nucleic acid is introduced into the host cell downstream of the gene of interest coding region, so as to produce a host cell; and
*d)* Expressing the protein of interest,
   wherein said method is not a method for treatment of the human or animal body practised on the human or animal body and wherein said method is not a process for modifying the germ line genetic identity of human beings, in accordance with claim 6.

It is disclosed a method not forming part of the present invention, wherein the said insert is ligated upstream of the gene coding sequence, for transcription at the 5' untranslated region (5'UTR).

It is disclosed a method not forming part of the present invention, wherein the said insert is ligated both upstream and downstream of the gene coding sequence, for transcription both at the 5'UTR and at the 3'UTR.

In an embodiment of the present invention's method, the said protein of interest is selected from the group consisting of a kinase, such as the Polo kinase protein or other enzyme proteins, a reporter protein (such as GFP and Luciferase proteins), an antibody or an antibody fragment protein, an hormone protein, a growth factor protein, a cytokine protein, a receptor protein, a structural protein and a viral protein, according to claim 7.

In another embodiment of the present invention's method, the said transformation, transduction, transfection or gene editing protocols include protocols known in the art that employ calcium chloride and heat-shock, viral particles, calcium phosphate, DEAE-dextran, electroporation, liposomes, non-liposomal lipids, dendrimers, guide RNAs and CRISPR/CAS9 enzymes or embryo microinjection, according to claim 8.

The present invention also refers to a kit for increasing the expression of a protein of interest comprising the compositions described in claim 2 prepared with the nucleic acid described in claim 1, or the genetic construct expression vectors described in claim 3, or the host cells described in claims 4-5, according to claim 9.

The present invention also refers to a nucleic acid with the 28-oligonucleotide base sequence SEQ ID nr.1. for use in a method for providing a therapy for a disease, through a gene editing method to introduce the said nucleic acid into host cells and increase the expression of a protein of interest, wherein the nucleic acid is introduced downstream of the gene of interest coding region, according to claim 10.

The present invention also refers to a nucleic acid with the 28-oligonucleotide base sequence SEQ ID nr.1. for use in the treatment of cancer, genetic or neurologic disorders, through a gene editing method to introduce the said nucleic acid into host cells and increase the expression of a protein of interest, wherein the nucleic acid is introduced downstream of the gene of interest coding region, according to claim 11.

Another aspect of the present invention refers to an *in vitro* method of lowering expression, substantially suppressing expression, or essentially shutting down expression of a protein of interest in a host cell comprising the step of: deleting or interfering with the nucleic acid with the 28-oligonucleotide base sequence of SEQ ID nr.1, according to claim 12.

The method, nucleic acid sequence, compositions, vectors, cell lines and kit of the present invention can be advantageously used as a new tool to boost protein production in biotechnology and/or biopharmaceutical applications, due to its ability to enhance protein production derived from a genetic construct, a gene expression or a reporter vector, in zebrafish, fruit fly or other model organisms, as well as in mammalian or human cells.

### Description of the invention

We found and characterized a new short non-coding 28 nucleotide sequence, herein named iPLUS - increase in Protein Levels Universal Sequence - and specified in SEQID nr. 1, that strongly increases protein production *in vitro* and *in vivo.*

Because it is short, this sequence can be obtained by chemical synthesis and can be easily inserted into expression vectors or into the genome of host cells. Surprisingly, it demonstrates a "universal" protein expression enhancer effect, which is effective in various types of host cells and for different types of proteins of interest. Furthermore, because it is non-coding, its effect is achieved without interfering with the gene coding region and with the protein produced.

The said iPLUS sequence (SEQID nr. 1) can be prepared by nucleotide synthesis method using the solid phase phosphoramidite nucleosides as described in the publication: Beaucage, SL; Iyer, RP (1992). "Advances in the Synthesis of Oligonucleotides by the Phosphoramidite Approach". Tetrahedron 48 (12): 2223. The synthesis can be performed on columns with solid support (controlled pore glass or polystyrene) functionalized with the first base of the 3 'end of each oligonucleotide. The preparation of the oligonucleotide follows after a number of synthesis cycles, each consisting of chemical reactions, typically four chemical reactions: i) release (detritylation), ii) coupling, iii) protection and iv) oxidation. In each cycle are added at the 5 'terminus of the growing chain, nucleotide residues corresponding to the desired sequence.

The nucleotides refer to nucleotides of natural or synthetic origin, with the hybridization capacity by base-pairing with complementary nucleotides, and may include, without limitation, DNA, RNA, and nucleotide analogues (e.g. nucleic acids with closed conformation, known as "locked nucleic acids" - LNA) nucleotides or without inter-nucleotide phosphodiester type linkages (e.g. peptide nucleic acid - PNA) and nucleic acids with tiodiester bonds, or the like for the same purpose.

A disclosure not forming part of the present invention relates to smaller truncated functional fragments of this sequence (SEQID nr. 1), between 4 and 27 nucleotides, most preferably 22 nucleotides, that can be inserted in the sequence of a gene, of an expression vector or of a reporter vector, to enhance protein production.

In an embodiment of the present invention, more than one copy of the nucleic acid of SEQID nr. 1 can be used in tandem repeats, in order to achieve higher fold-changes in protein production.

In another embodiment of the present invention, the nucleic acid with SEQID nr. 1 is used to generate a composition further comprising buffer, such as Tris-HCl, a divalent ion chelating agent, such as EDTA, or combinations thereof, for inserting the sequences into expression vectors or host cells.

The insertion of the sequence increasing protein production into expression or reporter vectors can be performed by DNA Ligase-mediated phosphodiester bonds between DNA fragments with "blunt ends".

In an embodiment of the present invention, a vector, we termed pLuc with similar features to the vector pmirGLO (from Promega), which expresses the reporter enzyme Luciferase, is linearized through SmaI digestion (1 hr at 37°C) and dephosphorylated with 2 µl of FastAP during 10 minutes at 37°C. Insert iPLUS nucleotide sequence (SEQID nr. 1) or a mutant form (iPLUS mutant),used as control, and the linearized pLuc are ligated, using 100 ng of pLuc and 19 ng of iPLUS or iPLUS mutant (5:1), at 22°C for 2h, in the presence of 5 units of T4 DNA Ligase.

In an embodiment of the sixth aspect of the invention, GFP-iPLUS according to the invention and GFP-iPLUS mutant sequences (control) are subcloned into SalI and KpnI restriction enzyme sites of the pUC19miniTOL expression vector by molecular biology methods employing compositions comprising the 28 nucleotide enhancing sequence.

Thus, in another embodiment of the present invention, the nucleic acid sequence (SEQID nr.1) in the insert composition can further comprise recognition sequences restriction enzymes upstream, downstream or at both ends of SEQ ID nr. 1, to provide "sticky-ends" for more efficient and oriented insert ligation.

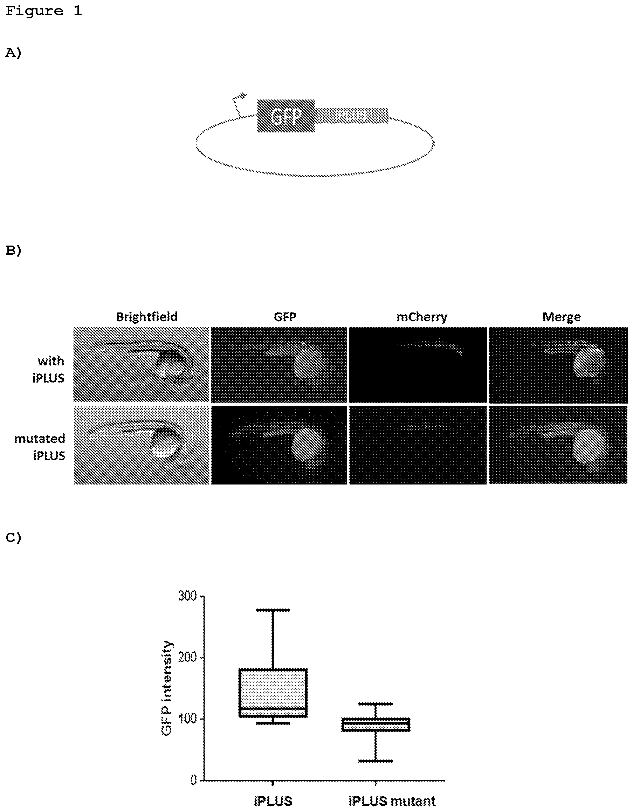

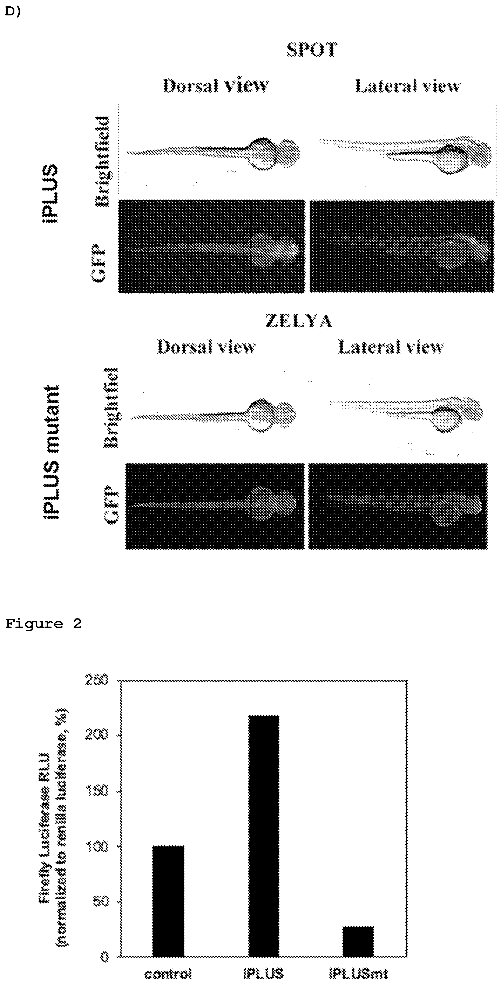

In another embodiment of the sixth aspect of the invention, the iPLUS is subcloned downstream of GFP and the resultant construct (puc19 miniTOL-GFP-iPLUS, Fig. 1A) is microinjected into zebrafish one cell stage embryos. A control construct is also made where the iPLUS sequence is mutated (puc19 miniTOL-GFP-iPLUSmt). Notably, GFP protein production is increased in zebrafish embryos microinjected with the iPLUS in comparison to the iPLUS mutant control, visualized by fluorescence microscopy (Fig. 1B). Quantification of protein level clearly shows that the iPLUS enhances GFP production in contrast to control (Fig. 1C). Zebrafish where GFP-iPLUS and GFP-iPLUS mutant were randomly inserted into the genome confirm that the iPLUS acts as an activator for protein production (Fig. 1D).

In another embodiment of the sixth aspect of the invention, the iPLUS sequence (SEQID nr.1) is sub-cloned downstream of the luciferase gene and the resultant construct (pLucDelSV40.iPLUS) is transfected into HeLa cells. A control construct was also designed where the iPLUS was mutated (pLucDelSV40.iPLUSmutant). Levels of Luciferase protein activity were quantified and results show that there is an eight-fold increase in cells transfected with the iPLUS in comparison to control (Fig. 2).

Thus, in the preferred embodiment of the present invention comprises the expression vectors in which iPLUS (SEQID nr.1) is inserted downstream of the encoded gene of interest (LUC or GFP in the former case).

The genetic constructs, such as expression vectors generated in the above mentioned manner constitute another aspect of the present invention, which refers to the genetic constructs and expression vectors according to claim 3 in which the SEQIDnr.1, or tandem repeats, are embodied.

Expression vectors in which iPLUS can be inserted may comprisea recombinant eukaryotic plasmid expression vector, a recombinant prokaryotic expression vector, a recombinant viral expression vector, a recombinant bacteriophage expression vector, a recombinant plant expression vector, a recombinant yeast mini-chromosome expression vector, a recombinant bacterial artificial chromosome expression vector, and a recombinant yeast expression plasmid vector.

The above mentioned genetic constructs or vectors with at least one copy of a nucleic acid with the 28-oligonucleotide base sequence SEQ ID nr.1. can also be used as reporters for testing the activity of the 28 nucleotide sequence, or smaller functional fragments of this sequence, to find modulators of its efficiency, including other genes, chemical compounds, or genetic modifications within host cells.

In another embodiment of the sixth aspect of the invention, competent bacteria are transformed with the ligation product comprising the plasmid expression vector embodying iPLUS (SEQID nr.1) by heat shock at 42°C for 1 min. Colonies harboring the iPLUS and iPLUS mutant are selected and plasmid DNA is obtained. Expression vector plasmids are then transfected into HeLa cells cultured in Dulbecco's modified Eagle's medium (DMEM 1x GlutaMAX, Gibco) supplemented with 10 % (v/v) Fetal Bovine Serum (FBS) (Gibco), with the Lipofectamine 3000 Reagent (from Thermo Fisher Scientific) according to a manufacturer's protocol.

In another embodiment of the sixth aspect of the invention, a mixture is microinjected into one cell stage zebrafish embryos containing 50 ng of pUC19miniTOL-GFP-iPLUS and pUC19miniTOL-GFP-iPLUS mutant control, plus 50ng of Tol2 and 0,1µL of PhenolRed (used as a dye to guide microinjection success). After microinjection, the embryos are placed in a E3 medium (28,67 g of NaCl, 1,26 g of KCl, 4,83 g of CaCl2.2H2O, 8,17 g of MgSO4.7H2O, 1,5 mL of methylene blue 0,1% filling until 1 L of H2O). After two hours post fertilization (hpf) the embryos are transferred to a PTU medium (600 mg of N-phenylthiourea dissolved in 200 mL of H2O). After 24 hpf, the embryos are bleached out and incubated at 28°C during the next first five days and then the embryos are transferred to small tanks with a low volume of water, being feed three times per day through a droplet mechanism. Embryos are then put in a Petri dish with agarose and PTU medium, and the chorion is removed. Tricaine MS222 (from Sigma) at concentration 0,168mg/ml is used to anesthetize the embryos according to good ethic practices. A stereomicroscope Leica M205 is used to screen embryos that expressed GFP and analyse the embryos microinjected with mCherry and pUC19-GFP-iPLUS or pUC19-GFP-iPLUS mutant control.

Thus, several types of host cells may be transfected with expression vectors comprising iPLUS for increased protein expression and, as such, other embodiments of the present invention refer to the host cell for increased expression of a protein of interest that comprise, stably or transiently, the expression vectors that integrate iPLUS (SEQID nr.1) according to claim 5.

Overall, the range of host cells generated by transfecting the iPLUS sequence (SEQID nr.1) may comprise a mammalian host cell (non-limiting examples of which include a HeLa cell, but also a Chinese Hamster Ovary cell, a COS cell, a Vero cell, an SP2/0 cell, an NS/0 myeloma cell, a human embryonic kidney 293 cell, a baby hamster kidney cell, a human B cell, a human T cell Jurkat, a neuronal cell, a CV-I /EBNA cell, an L cell, a 3T3 cell, an HEPG2 cell, and an MDCK cell), an insect host cell (non-limiting example of such includes Drosophila cells), a fish host cell (non-limiting example includes zebrafish one cell embryo), a plant host cell (non-limiting example includes Nicotania tabacum, Arabidopsis, Lactuca sativa, protoplasts, suspension cells) a fungal host cell (non-limiting examples of which include Aspergillus, Neurospora, Saccharomyces, Pichia, Hansenula, Schizosaccharomyces, Kluyveromyces, Yarrowia, and Candida cells), an algal host cell (non-limiting example of which includes Synechocystis and other Cyanobacteria cells), a nematode host cell, a protozoan host cell, and a prokaryotic host cell (non-limiting examples of which include Escherichia coli, *Erwinia chrysanthemi* and *Clostridium histolyticum cells*)*.*

Aside from host cells embodying an expression vector comprising iPLUS (SEQID nr.1) another embodiment of the present invention refers to host cells according to claim 4 comprising at least one exogenous copy of the nucleic acid with sequence SEQ ID nr. 1, not necessarily integrated into an expression vector, which is introduced downstream of a gene of interest for example through gene editing protocols known in the art, and remains under the control of the native gene regulator elements.

According to the above mentioned embodiments, a sixth aspect of the present invention refers to a method for increasing the expression of a protein of interest in a host cell characterized by comprising the steps of:
*a)* Synthesizing a nucleic acid for increasing the expression of a protein of interest comprising at least one copy of the 28-oligonucleotide with the base sequence of SEQ ID nr. 1 and preparing compositions comprising said nucleic acid, a buffer such as Tris-HCl, a divalent ion chelating agent, such as EDTA, and combinations thereof;
*b)* Contacting the nucleic acid composition with a linearized expression vector encoding for a protein of interest and ligating the nucleic acid downstream of the gene coding sequence, for transcription at the 3' untranslated region (3'UTR) through incubation with a DNA Ligase in the appropriate buffer and temperature conditions, so as to obtain an expression vector comprising at least one copy of the nucleic acid with SEQ ID nr. 1 downstream of the gene of interest coding region;
*c)* Introducing the said expression vectors or the nucleic acid for increasing the expression of a protein of interest comprising at least one copy of the 28-oligonucleotide with the base sequence of SEQ ID nr. 1 into a host cell, trough transient or stable transformation, transduction, transfection or trough gene editing protocols, wherein said nucleic acid is introduced into the host cell downstream of the gene of interest coding region, so as to produce a host cell ;and
*d)* Expressing the protein of interest,
   wherein said method is not a method for treatment of the human or animal body practiced on the human or animal body and wherein said method is not a process for modifying the germ line genetic identity of human beings.

Also, according to the herein described embodiments, the said protein of interest may be selected from the group consisting of a kinase, such as the Polo kinase protein or other enzyme proteins, a reporter protein (such as GFP and Luciferase proteins), as well as other common recombinant proteins such as an antibody or an antibody fragment protein, an hormone protein, a growth factor protein, a cytokine protein, a receptor protein, a structural protein and a viral protein.

According to the herein described embodiments, in the present invention's method, the said transformation, transduction, transfection or gene editing protocols include protocols known in the art that employ calcium chloride competent bacteria and heat-shock, liposomes such as the Lipofectamine 3000 reagent, direct, as well as other protocols known in the art comprising viral particles, calcium phosphate, DEAE-dextran, electroporation, non-liposomal lipids, dendrimers and guide RNAs and CRISPR/CAS9 enzymes.

According to the herein described embodiments, the present invention also refers to a kit for increasing the expression of a protein of interest comprising the compositions described in claim 2 prepared with the iPLUS nucleic acids with SEQID nr.1 described in claim 1, or the genetic construct expression vectors described in claim 3, or the host cells described in claims 4-5.

Due to the fact that iPLUS is a short sequence, it is amenable to integration into host cell lines, including embryo cells, through gene editing protocols known in the art, and thus the present invention also refers to a nucleic acid with the 28-oligonucleotide base sequence SEQ ID nr.1. for use in a method for providing therapy of a disease such as, but not limited to, cancer, genetic, pancreatic or neurologic disorders, through a gene editing method to introduce the exogenous nucleic acid into host cells and increase the expression of a protein of interest, wherein the nucleic acid is introduced downstream of the gene of interest coding region.

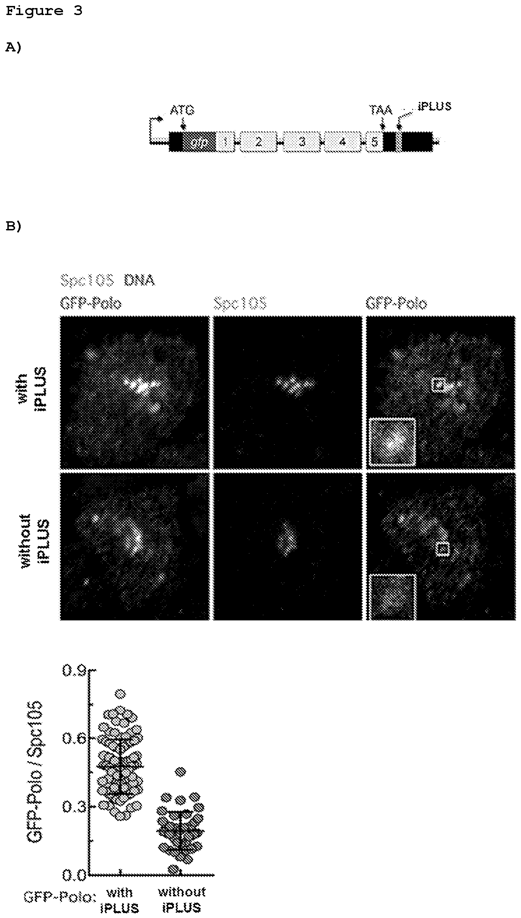

In one embodiment of the present invention, the polo kinase protein levels were quantified in transgenic flies by confocal microscopy. Transgenic flies carrying pW8-gfp-polo kinase protein with either iPLUS or a deleted iPLUS sequence (gfp-polo-Del-iPLUS are obtained by injection of ww1118 embryos with the respective transgenes (Fig. 3A). Selection of transgenic lines with the transgene on the second chromosome was performed by mating the transgenic flies with a strain carrying dominant markers and a balancer chromosome, w1118; Sco/SM6. These are then mated with w1118; If/CyO; MKRS/TM6B and w1118; If/CyO; polo9/TM6C to generate the w1118; gfp-polo-Del-iPLUS; polo9/TM6B line (herein referred as Del-iPLUS transgenic fly strain). Dissected larvae brains from each strain are placed in fresh 50 µM colchicine in PBS for 1h30 at 25°C and then fixated for 5 min with 1.8% formaldehyde and 45% acetic acid. Brain squashes are incubated with absolute ethanol for 10 min, then 10 min with PBS 0.1% Triton X-100 and a 10 min wash with PBS. Fixated brains are blocked with 10% FBS in PBS-T 0.05% for 1h at RT and then incubated overnight at 4°C with the following antibodies: rat anti-Spc105 (1:150), mouse anti-Polo (1:10, MA294), rabbit anti-P-Aurora (1:500, Rockland, Limerik, PA), rabbit anti-Thr676-P-Mps1 (1:10000) and ginea pig anti-total Mps1 (1:250, Gp15 RRID:AB 2567774). This is followed by three 5 minutes washes in PBS 0.05% Tween20 and incubation with the following secondary antibodies (Thermo Fischer) diluted 1:250 in PBS 0.05% Tween20 for 2h at RT: goat anti-rat AlexaFluor 647, goat anti-mouse AlexaFluor 488, goat anti-rabbit AlexaFluor 568 and goat anti-ginea pig AlexaFluor 568. Image acquisition is performed using a Laser Scanning confocal microscope Leica TCS SP5 II and the LAS 2.6 software (Leica Microsystems, Germany). Results of Polo protein expression *in vivo* clearly show a strong reduction in protein production in transgenic flies without iPLUS (Fig. 3B and 3C).

Thus, another aspect of the present invention refers to an *in vitro* method of lowering expression, substantially suppressing expression, or essentially shutting down expression of a protein of interest in a host cell comprising a step of deleting or interfering with the nucleic acid with the 28-oligonucleotide base sequence of SEQ ID nr.1.

The nucleic acid sequence, compositions, vectors, host cell lines, methods, kit and uses of the present invention can be advantageously used as a new tool to boost protein production in biotechnology and/or biopharmaceutical applications, due to its ability to enhance protein production derived from a genetic construct, a gene expression or a reporter vector, in zebrafish, fruit fly or other model organisms, as well as in mammalian or human cells.

### Brief Description of the Figures

**Figure 1****:** Embodiment of the sixth aspect of the invention in which iPLUS (SEQID nr.1) increases GFP expression in zebrafish. A) Schematic representation of the construct used to generate the transgenic fish with the iPLUS inserted in the 3'UTR of GFP (puc19 miniTOL-GFP-iPLUS)). B) The iPLUS activates GFP expression in comparison to the mutated iPLUS. Microinjection of puc19 miniTOL-GFP-iPLUS and puc19 miniTOL-GFP-iPLUSmt with mylz promotor mCherry in embryos at one cell stage. Images were acquired at 24h stage. C) Quantification of iPLUS vs iPLUS mutant GFP expression. Analysis of GFP intensity of 30 embryos at 24h stage, in each condition. **D)** Intense GFP expression in iPLUS founder Spot in comparison with the iPLUS mutant founder Zelya. Images were acquired at 48h stage in dorsal and ventral view.
**Figure 2****:** Embodiment of the sixth aspect of the invention in which Luciferase activity is increased in HeLa cells transfected with iPLUS (SEQID nr. 1). Percentage of luciferase/Renilla (RLU) with different plasmids (pLucDelSV40, pLucDelSV40.iPLUS and pLucDelSV40.iPLUSmutant) in HeLa cells. The expression of Renilla and Luciferase is measured by the Dual Luciferase Reporter Assay (Promega) in a plate reader (Synergy 2).
**Figure 3****:** Embodiment in which iPLUS (SEQID nr.1) was deleted to decrease gene expression. A) Schematic representation of the transgene constructed to generate the transgenic flies wherein the iPLUS sequence is comprised at the 3'UTR, downstream of the polo gene. The arrow at the beginning of the transgene represents the endogenous polo promoter, the grey boxes represent the five polo exons, the black boxes represent the 5' and 3'UTRs and the gfp box represents the gfp coding sequence inserted in frame with polo initiation codon. The hatched box in the 3'UTR represents the iPLUS sequence, which was deleted from the 3'UTR sequence. B) GFP-Polo expression is decreased in flies with iPLUS deletion, as assessed by quantification of immunofluorescence of GFP-Polo in dividing neuroblasts; with Spc105 used as a kinetochore marker.

In conclusion, the iPLUS, a 28 nucleotide nucleic acid sequence, is a new activator of protein production in vivo and in vitro in different model organisms comprising fruit fly, zebrafish and in human cells. The iPLUS can be easily manipulated by use of compositions, methods and kits to be used as an effective tool to boost production of proteins with in biotechnology and/or biopharmaceutical applications.

Other variations and embodiments of the invention described herein will now be apparent to those skilled in the art, and all such variants and alternative embodiments of the invention are intended to be encompassed within the foregoing description and the claims that follow.

### SEQUENCE LISTING

**SEQ ID nr.1:** 5'-AATTTATTTGTTTTTGCCCCTTCCCCTT-3'
Porto, October 9^{th}, 2019

## Claims

1. Nucleic acid consisting of the 28-oligonucleotide with the base sequence of SEQ ID nr.1 or consisting of tandem repeats of the 28-oligonucleotide with the base sequence of SEQ ID nr. 1.

2. Composition for increasing the expression of a protein of interest comprising:
(i) a nucleic acid as described in claim 1, or a nucleic acid consisting of the sequence SEQ ID nr. 1 or consisting of tandem repeats of the sequence SEQ ID nr. 1 having restriction enzyme recognition sequences upstream, downstream or at both ends of the nucleic acid described in claim 1;
(ii) a buffer such as Tris-HCl;
(iii) a divalent ion chelating agent, such as EDTA; and
(iv) combinations thereof.

3. Genetic construct for increasing the expression of a protein of interest comprising at least one copy of the nucleic acid with SEQ ID nr.1, as described in claim 1, downstream of the gene of interest coding region, wherein the genetic construct for increasing recombinant protein expression is an expression vector selected from the group consisting of a recombinant eukaryotic plasmid expression vector, a recombinant viral expression vector, a recombinant bacteriophage expression vector, a recombinant yeast mini-chromosome expression vector, a recombinant plant vector, a recombinant bacterial artificial chromosome expression vector, and a recombinant yeast expression plasmid vector.

4. Host cell for increased expression of a protein of interest comprising at least one exogenous copy of the nucleic acid described in claim 1, introduced downstream of the gene of interest coding region for example through gene editing, wherein the host cell is selected from the group consisting of a mammalian host cell, such as a HeLa cell, a Chinese Hamster Ovary cell, a COS cell, a Vero cell, an SP2/0 cell, an NS/0 myeloma cell, a human embryonic kidney 293 cell, a baby hamster kidney cell, a human B cell, a human T Cell Jurkat, a neuronal cell, a CV-I /EBNA cell, an L cell, a 3T3 cell, an HEPG2 cell, and an MDCK cell; a fish host cell, such as zebrafish; a plant host cell, such as Nicotania tabacum, Arabidopsis, Lactuca sativa, protoplasts andsuspension cells; a fungal host cell, such as Aspergillus, Neurospora, Saccharomyces, Pichia, Hansenula, Schizosaccharomyces, Kluyveromyces, Yarrowia, and Candida cells; an algal host cell, such as Synechocystis and other Cyanobacteria cells; a nematode host cell; and a protozoan host cell; and wherein the host cell is not a human germ cell.

5. Host cell for increased expression of a protein of interest comprising, stably or transiently, the genetic constructs or expression vectors described in claim 3 wherein the host cell is selected from the group consisting of a mammalian host cell, such as a HeLa cell, a Chinese Hamster Ovary cell, a COS cell, a Vero cell, an SP2/0 cell, an NS/0 myeloma cell, a human embryonic kidney 293 cell, a baby hamster kidney cell, a human B cell, a human T Cell Jurkat, a neuronal cell, a CV-I /EBNA cell, an L cell, a 3T3 cell, an HEPG2 cell, and an MDCK cell; a fish host cell, such as zebrafish; a plant host cell, such as Nicotania tabacum, Arabidopsis, Lactuca sativa, protoplasts, and suspension cells; a fungal host cell, such as Aspergillus, Neurospora, Saccharomyces, Pichia, Hansenula, Schizosaccharomyces, Kluyveromyces, Yarrowia, and Candida cells; an algal host cell, such asSynechocystis and other Cyanobacteria cells a nematode host cell; a protozoan host cell; and a prokaryotic host cell, such as *Escherichia coli, Erwinia chrysanthemi* and *Clostridium histolyticum* cells; and wherein the host cell is not a human germ cell.

6. A method for increasing the expression of a protein of interest in a host cell comprising the steps of:
***a)*** Synthesizing a nucleic acid for increasing the expression of a protein of interest comprising at least one copy of the 28-oligonucleotide with the base sequence of SEQ ID nr.1 and preparing compositions comprising said nucleic acid, a buffer such as Tris-HCl, a divalent ion chelating agent, such as EDTA, and combinations thereof;
***b)*** Contacting the nucleic acid composition with a linearized expression vector encoding for a protein of interest and ligating the nucleic acid downstream of the gene coding sequence, for transcription at the 3' untranslated region (3'UTR) through incubation with a DNA Ligase in the appropriate buffer and temperature conditions, so as to obtain an expression vector comprising at least one copy of the nucleic acid with SEQ ID nr. 1 downstream of the gene of interest coding region;
***c)*** Introducing the said expression vector or the nucleic acid for increasing the expression of a protein of interest comprising at least one copy of the 28-oligonucleotide with the base sequence of SEQ ID nr. 1 into a host cell, through transient or stable transformation, transduction, transfection or through gene editing protocols, wherein said nucleic acid is introduced into the host cell downstream of the gene of interest coding region, so as to produce a host cell such as the host cell comprising at least one exogenous copy of the nucleic acid for increasing the expression of a protein of interest comprising at least one copy of the 28-oligonucleotide with the base sequence of SEQ ID nr. 1, introduced for example through gene editing; and
***d)*** Expressing the protein of interest,
wherein said method is not a method for treatment of the human or animal body practised on the human or animal body and wherein said method is not a process for modifying the germ line genetic identity of human beings.

7. Method according to claim 6 wherein the said protein of interest is selected from the group consisting of a kinase, such as the Polo kinase protein or other enzyme proteins, a reporter protein , such as GFP and Luciferase proteins , an antibody or an antibody fragment protein, a hormone protein,
a growth factor protein, a cytokine protein, a receptor protein, a structural protein and a viral protein.

8. Method according to claim 6 wherein the said transformation, transduction, transfection or gene editing protocols employ calcium chloride and heat-shock, viral particles, calcium phosphate, DEAE-dextran, electroporation, liposomes, non-liposomal lipids, dendrimers, guide RNAs and CRISPR/CAS9 enzymes or embryo microinjection at one cell stage.

9. A kit for increasing the expression of a protein of interest comprising the compositions described in claim 2 prepared with the nucleic acid described in claim 1, or the genetic construct expression vectors described in claim 3, or the host cells described in claims 4-5.

10. A nucleic acid with the 28-oligonucleotide base sequence SEQ ID nr.1. for use in a method for providing a therapy for a disease, through a gene editing method to introduce the said nucleic acid into host cells and increase the expression of a protein of interest, wherein the nucleic acid is introduced downstream of the gene of interest coding region.

11. A nucleic acid with the 28-oligonucleotide base sequence SEQ ID nr.1. for use in the treatment of cancer, genetic or neurologic disorders, through a gene editing method to introduce the said nucleic acid into host cells and increase the expression of a protein of interest, wherein the nucleic acid is introduced downstream of the gene of interest coding region.

12. An *in vitro* method of lowering expression, substantially suppressing expression, or essentially shutting down expression of a protein of interest in a host cell comprising the step of: deleting or interfering with the nucleic acid with the 28-oligonucleotide base sequence of SEQ ID nr.1.

## Patentansprüche

1. Nukleinsäure, bestehend aus dem 28-Oligonukleotid mit der Basensequenz von SEQ ID Nr. 1 oder bestehend aus Tandemwiederholungen des 28-Oligonukleotids mit der Basensequenz von SEQ ID Nr. 1.

2. Zusammensetzung zur Steigerung der Expression eines Proteins von Interesse, umfassend:
(i) eine Nukleinsäure wie in Anspruch 1 beschrieben, oder eine Nukleinsäure, die aus der Sequenz SEQ ID Nr. 1 besteht oder aus Tandem-Wiederholungen der Sequenz SEQ ID Nr. 1 mit Restriktionsenzym-Erkennungssequenzen stromaufwärts, stromabwärts oder an beiden Enden der in Anspruch 1 beschriebenen Nukleinsäure besteht;
(ii) einen Puffer wie Tris-HCl;
(iii) einen Chelatbildner für zweiwertige Ionen, wie EDTA, und
(iv) Kombinationen davon.

3. Genetisches Konstrukt zur Steigerung der Expression eines Proteins von Interesse, umfassend mindestens eine Kopie der Nukleinsäure mit der SEQ ID Nr. 1, wie in Anspruch 1 beschrieben, stromabwärts der kodierenden Region des Gens von Interesse, wobei das genetische Konstrukt zur Steigerung der rekombinanten Proteinexpression ein Expressionsvektor ist, der aus der Gruppe ausgewählt ist, die aus einem rekombinanten eukaryotischen Plasmid-Expressionsvektor, einem rekombinanten viralen Expressionsvektor, einem rekombinanten Bakteriophagen-Expressionsvektor, einem rekombinanten Hefe-Mini-Chromosom-Expressionsvektor, einem rekombinanten Pflanzenvektor, einem rekombinanten bakteriellen künstlichen Chromosom-Expressionsvektor und einem rekombinanten Hefe-Expressionsplasmidvektor besteht.

4. Wirtszelle zur Steigerung der Expression eines Proteins von Interesse, umfassend mindestens eine exogene Kopie der in Anspruch 1 beschriebenen Nukleinsäure, die stromabwärts der kodierenden Region des Gens von Interesse, beispielsweise durch Gen-Editing, eingeführt wurde, wobei die Wirtszelle aus der Gruppe ausgewählt ist bestehend aus einer Säugetier-Wirtszelle, wie einer HeLa-Zelle, einer Ovarialzelle des Chinesischen Hamsters, einer COS-Zelle, einer Vero-Zelle, einer SP2/0-Zelle, einer NS/0-Myelomzelle, einer menschlichen embryonalen Nierenzelle 293, einer Babyhamsternierenzelle, einer menschlichen B-Zelle, einer menschlichen T-Zelle Jurkat, einer neuronalen Zelle, einer CV-I /EBNA-Zelle, einer L-Zelle, einer 3T3-Zelle, einer HEPG2-Zelle und einer MDCK-Zelle; eine Fisch-Wirtszelle, wie z. B. Zebrafisch; eine Pflanzen-Wirtszelle, wie z. B. Nicotania tabacum, Arabidopsis, Lactuca sativa, Protoplasten und Suspensionszellen; eine Pilz-Wirtszelle, wie z. B. Aspergillus-, Neurospora-, Saccharomyces-, Pichia-, Hansenula-, Schizosaccharomyces-, Kluyveromyces-, Yarrowia- und Candida-Zellen; eine Algenwirtszelle, wie Synechocystis und andere Cyanobakterienzellen; eine Nematodenwirtszelle; und eine Protozoenwirtszelle; und wobei die Wirtszelle keine menschliche Keimzelle ist.

5. Wirtszelle zur Steigerung der Expression eines Proteins von Interesse, die stabil oder transient die in Anspruch 3 beschriebenen genetischen Konstrukte oder Expressionsvektoren umfasst, wobei die Wirtszelle ausgewählt ist aus der Gruppe bestehend aus einer Säugetier-Wirtszelle, wie einer HeLa-Zelle, einer Ovarialzelle des Chinesischen Hamsters, einer COS-Zelle, einer Vero-Zelle, einer SP2/0-Zelle, einer NS/0-Myelomzelle, einer menschlichen embryonalen Nierenzelle 293, einer Babyhamsternierenzelle, einer menschlichen B-Zelle, einer menschlichen T-Zelle Jurkat, einer neuronalen Zelle, einer CV-I /EBNA-Zelle, einer L-Zelle, einer 3T3-Zelle, einer HEPG2-Zelle und einer MDCK-Zelle; eine Fisch-Wirtszelle, wie z. B. Zebrafisch; eine Pflanzen-Wirtszelle, wie z. B. Nicotania tabacum, Arabidopsis, Lactuca sativa, Protoplasten und Suspensionszellen; eine Pilz-Wirtszelle, wie z. B. Aspergillus-, Neurospora-, Saccharomyces-, Pichia-, Hansenula-, Schizosaccharomyces-, Kluyveromyces-, Yarrowia- und Candida-Zellen; eine Algenwirtszelle, wie Synechocystis und andere Cyanobakterienzellen; eine Nematodenwirtszelle; eine Protozoenwirtszelle; und eine prokaryotische Wirtszelle, wie *Escherichia coli, Erwinia chrysanthemi* und *Clostridium* histolyticum-Zellen; und wobei die Wirtszelle keine menschliche Keimzelle ist.

6. Verfahren zur Steigerung der Expression eines Proteins von Interesse in einer Wirtszelle, umfassend die folgenden Schritte:
***a)*** Synthese einer Nukleinsäure zur Steigerung der Expression eines Proteins von Interesse, die mindestens eine Kopie des 28-Oligonukleotids mit der Basensequenz von SEQ ID Nr. 1 umfasst, und Herstellung von Zusammensetzungen, die die genannte Nukleinsäure, einen Puffer, wie Tris-HCl, einen Chelatbildner für zweiwertige Ionen, wie EDTA, und Kombinationen davon umfassen;
***b)*** Kontaktieren der Nukleinsäurezusammensetzung mit einem linearisierten Expressionsvektor, der für ein Protein von Interesse kodiert, und Ligieren der Nukleinsäure stromabwärts der Genkodierungssequenz zur Transkription an der 3'-untranslatierten Region (3'UTR) durch Inkubation mit einer DNA-Ligase in den geeigneten Puffer- und Temperaturbedingungen, um einen Expressionsvektor zu erhalten, der mindestens eine Kopie der Nukleinsäure mit SEQ ID Nr. 1 stromabwärts der Genkodierungsregion von Interesse umfasst;
***c)*** Einführen des genannten Expressionsvektors oder der Nukleinsäure zur Steigerung der Expression eines Proteins von Interesse, das mindestens eine Kopie des 28-Oligonukleotids mit der Basensequenz von SEQ ID Nr. 1 umfasst, in eine Wirtszelle durch transiente oder stabile Transformation, Transduktion, Transfektion oder durch Gen-Editing-Protokolle, wobei die genannte Nukleinsäure in die Wirtszelle stromabwärts der kodierenden Region des Gens von Interesse eingeführt wird, um eine Wirtszelle zu erzeugen, wie zum Beispiel die Wirtszelle, die mindestens eine exogene Kopie der Nukleinsäure zur Steigerung der Expression eines Proteins von Interesse umfasst, das mindestens eine Kopie des 28-Oligonukleotids mit der Basensequenz von SEQ ID Nr. 1 umfasst, die beispielsweise durch Gen-Editing eingeführt wurde; und
***d)*** Expression des Proteins von Interesse,
wobei es sich beim genannten Verfahren nicht um ein Verfahren zur Behandlung des menschlichen oder tierischen Körpers handelt, das am menschlichen oder tierischen Körper durchgeführt wird, und wobei es sich beim genannten Verfahren nicht um ein Verfahren zur Veränderung der genetischen Identität der Keimbahn von menschlichen Wesen handelt.

7. Verfahren nach Anspruch 6, wobei das genannte Protein von Interesse aus der Gruppe ausgewählt ist, die aus einer Kinase, wie dem Polo-Kinase-Protein oder anderen Enzymproteinen, einem Reporterprotein, wie GFP- und Luciferase-Proteinen, einem Antikörper oder einem Antikörperfragment-Protein, einem Hormonprotein, einem Wachstumsfaktor-Protein, einem Zytokin-Protein, einem Rezeptor-Protein, einem Strukturprotein und einem viralen Protein besteht.

8. Verfahren nach Anspruch 6, bei dem die genannten Transformations-, Transduktions-, Transfektions- oder Gen-Editierungsprotokolle Calciumchlorid und Hitzeschock, Viruspartikel, Calciumphosphat, DEAE-Dextran, Elektroporation, Liposomen, nicht-liposomale Lipide, Dendrimere, Leit-RNAs und CRISPR/CAS9-Enzyme oder Embryo-Mikroinjektion im Ein-Zellstadium verwenden.

9. Kit zur Steigerung der Expression eines Proteins von Interesse, umfassend die in Anspruch 2 beschriebenen Zusammensetzungen, die mit der in Anspruch 1 beschriebenen Nukleinsäure oder den in Anspruch 3 beschriebenen genetischen Konstrukt-Expressionsvektoren oder den in den Ansprüchen 4-5 beschriebenen Wirtszellen hergestellt wurden.

10. Nukleinsäure mit der 28-Oligonukleotid-Basensequenz SEQ ID Nr. 1 zur Verwendung in einem Verfahren zur Bereitstellung einer Therapie für eine Krankheit durch ein Gen-Editierverfahren, um die genannte Nukleinsäure in Wirtszellen einzuführen und die Expression eines Proteins von Interesse zu steigern, wobei die Nukleinsäure stromabwärts der kodierenden Region des Gens von Interesse eingeführt wird.

11. Nukleinsäure mit der 28-Oligonukleotid-Basensequenz SEQ ID Nr. 1 zur Verwendung bei der Behandlung von Krebs, genetischen oder neurologischen Störungen durch ein Gen-Editierverfahren, um die genannte Nukleinsäure in Wirtszellen einzuführen und die Expression eines Proteins von Interesse zu steigern, wobei die Nukleinsäure stromabwärts der kodierenden Region des Gens von Interesse eingeführt wird.

12. *In vitro*-Verfahren zur Verringerung der Expression, zur wesentlichen Unterdrückung der Expression oder zur wesentlichen Abschaltung der Expression eines Proteins von Interesse in einer Wirtszelle, umfassend den Schritt: Deletion oder Interferenz mit der Nukleinsäure mit der 28-Oligonukleotid-Basensequenz von SEQ ID Nr. 1.

## Revendications

1. Acide nucléique constitué de la séquence des bases à 28 oligonucléotides de la SÉQ ID N° 1 ou constitué de répétitions en tandem de la séquence des bases à 28 oligonucléotides de la SÉQ ID N° 1.

2. Composition destinée à augmenter l'expression d'une protéine d'intérêt comprenant :
(i) un acide nucléique tel que décrit dans la revendication 1, ou un acide nucléique constitué de la séquence SÉQ ID N° 1 ou constitué de répétitions en tandem de la séquence SÉQ ID N° 1 ayant des séquences de reconnaissance d'enzymes de restriction en amont, en aval ou aux deux extrémités de l'acide nucléique décrit dans la revendication 1,
(ii) un tampon tel que Tris-HCl,
(iii) un agent chélateur des ions divalents, tel qu'EDTA, et
(iv) des combinaisons de ceux-ci.

3. Construction génétique destinée à augmenter l'expression d'une protéine d'intérêt comprenant au moins une copie de l'acide nucléique avec la SÉQ ID N° 1, tel que décrit dans la revendication 1, en aval du gène de la région codante d'intérêt, dans laquelle la construction génétique destinée à augmenter l'expression des protéines recombinantes est un vecteur d'expression choisi parmi le groupe constitué d'un vecteur d'expression recombinant de type plasmide eucaryote, d'un vecteur d'expression viral recombinant, d'un vecteur d'expression recombinant de type bactériophage, d'un vecteur d'expression recombinant de minichromosome de levure, d'un vecteur d'expression recombinant de plante, d'un vecteur d'expression recombinant de chromosome artificiel bactérien et d'un vecteur d'expression recombinant de plasmide de levure.

4. Cellule hôte pour l'expression accrue d'une protéine d'intérêt comprenant au moins une copie exogène de l'acide nucléique décrit dans la revendication 1, introduit en aval du gène de la région codante d'intérêt, par exemple par édition génomique, dans laquelle la cellule hôte est choisie parmi le groupe constitué d'une cellule hôte de mammifère, telle qu'une cellule HeLa, d'une cellule ovarienne de hamster, d'une cellule COS, d'une cellule Vero, d'une cellule SP2/0 , d'une cellule de myélome NS/0, d'une cellule rénale embryonnaire humaine 293, d'une cellule rénale de bébé hamster, d'une cellule B humaine, d'une cellule T Jurkat humaine, d'une cellule neuronale, d'une cellule CV-I /EBNA, d'une cellule L, d'une cellule 3T3, d'une cellule HEPG2 et d'une cellule MDCK, d'une cellule hôte de poisson, tel qu'un poisson zèbre, d'une cellule hôte de plante, telle que Nicotania tabacum, Arabidopsis, Lactuca sativa, de protoplastes et de cellules en suspension, d'une cellule hôte mycosique, telle que des cellules Aspergillus, Neurospora, Saccharomyces, Pichia, Hansenula, Schizosaccharomyces, Kluyveromyces, Yarrowia et Candida, d'une cellule hôte alguaire telle que Synechocystis et d'autres cellules Cyanobacteria, d'une cellule hôte de nématode et d'une cellule hôte de protozoaire, et dans laquelle la cellule hôte n'est pas une cellule germinale humaine.

5. Cellule hôte pour l'expression accrue d'une protéine d'intérêt comprenant, de manière stable ou transitoire, les constructions génétiques ou les vecteurs d'expression décrits dans la revendication 3, dans laquelle la cellule hôte est choisie parmi le groupe constitué d'une cellule hôte de mammifère, telle qu'une cellule HeLa, d'une cellule ovarienne de hamster, d'une cellule COS, d'une cellule Vero, d'une cellule SP2/0, d'une cellule de myélome NS/0, d'une cellule rénale embryonnaire humaine 293, d'une cellule rénale de bébé hamster, d'une cellule B humaine, d'une cellule T Jurkat humaine, d'une cellule neuronale, d'une cellule CV-I /EBNA, d'une cellule L, d'une cellule 3T3, d'une cellule HEPG2 et d'une cellule MDCK, d'une cellule hôte de poisson, tel qu'un poisson zèbre, d'une cellule hôte de plante, telle que Nicotania tabacum, Arabidopsis, Lactuca sativa, de protoplastes et de cellules en suspension, d'une cellule hôte mycosique, telle que des cellules Aspergillus, Neurospora, Saccharomyces, Pichia, Hansenula, Schizosaccharomyces, Kluyveromyces, Yarrowia et Candida, d'une cellule hôte alguaire telle que Synechocystis et d'autres cellules Cyanobacteria, d'une cellule hôte de nématode, d'une cellule hôte de protozoaire et d'une cellule hôte procaryote, telle que des cellules Escherichia coli, Erwinia chrysanthemi et Clostridium histolyticum, et dans laquelle la cellule hôte n'est pas une cellule germinale humaine.

6. Procédé pour augmenter l'expression d'une protéine d'intérêt dans une cellule hôte, comprenant les étapes consistant à :
a) synthétiser un acide nucléique pour augmenter l'expression d'une protéine d'intérêt comprenant au moins une copie de la séquence des bases à 28 oligonucléotides de la SÉQ ID N° 1 et préparer des compositions comprenant ledit acide nucléique, un tampon tel que Tris-HCl, un agent chélateur des ions divalents, tel qu'EDTA, et des combinaisons de ceux-ci,
b) mettre en contact la composition d'acide nucléique avec un vecteur d'expression linéarisé codant pour une protéine d'intérêt et ligaturer l'acide nucléique en aval de la séquence de codage génétique, pour une transcription au niveau de la région non traduite 3' (3'UTR) par incubation avec une ADN ligase dans le tampon et des conditions de température appropriés, de manière à obtenir un vecteur d'expression comprenant au moins une copie de l'acide nucléique avec la SÉQ ID N° 1 en aval du gène de la région codante d'intérêt,
c) introduire ledit vecteur d'expression ou l'acide nucléique pour augmenter l'expression d'une protéine d'intérêt comprenant au moins une copie de la séquence des bases à 28 oligonucléotide de la SÉQ ID N° 1 dans une cellule hôte, par des protocoles transitoires ou stables de transformation, de transduction, de transfection ou d'édition génomique, dans lequel ledit acide nucléique est introduit dans la cellule hôte en aval du gène de la région codante d'intérêt, de manière à produire une cellule hôte telle que la cellule hôte comprenant au moins une copie exogène de l'acide nucléique destiné à augmenter l'expression d'une protéine d'intérêt comprenant au moins une copie de la séquence des bases à 28 oligonucléotides de la SÉQ ID N° 1, introduit par exemple par édition génomique, et
d) exprimer la protéine d'intérêt, dans lequel ledit procédé n'est pas un procédé destiné au traitement du corps humain ou animal effectué sur le corps humain ou animal et dans lequel ledit procédé n'est pas un processus destiné à modifier l'identité génétique de lignée germinale d'êtres humains.

7. Procédé selon la revendication 6, dans lequel ladite protéine d'intérêt est choisie parmi le groupe constitué d'une kinase, telle que la protéine Polo kinase ou d'autres protéines d'enzyme, une protéine de rapporteur, telle que des protéines GFP et luciférase, une protéine d'anticorps ou de fragment d'anticorps, une protéine d'hormone, une protéine de facteur de croissance, une protéine de cytokine, une protéine de récepteur, une protéine structurale et une protéine virale.

8. Procédé selon la revendication 6, dans lequel lesdits protocoles de transformation, de transduction, de transfection ou d'édition génomique utilisent du chlorure de calcium et un choc thermique, des particules virales, du phosphate de calcium, du DEAE-dextrane, une électroporation, des liposomes, des lipides non liposomaux, des dendrimères, des ARN de guidage et des enzymes CRISPR/CAS9 ou une microinjection d'embryon à une phase cellulaire.

9. Kit pour augmenter l'expression d'une protéine d'intérêt comprenant les compositions décrites dans la revendication 2 préparées avec l'acide nucléique décrit dans la revendication 1, ou les vecteurs d'expression de construction génétique décrits dans la revendication 3, ou les cellules hôtes décrites dans les revendications 4 à 5.

10. Acide nucléique avec la séquence des bases à 28 oligonucléotides de la SÉQ ID N° 1 pour une utilisation dans un procédé destiné à fournir une thérapie pour une maladie, par un procédé d'édition génomique pour introduire le dit acide nucléique dans des cellules hôtes et augmenter l'expression d'une protéine d'intérêt, dans lequel l'acide nucléique est introduit en aval du gène de la région codante d'intérêt.

11. Acide nucléique avec la séquence des bases à 28 oligonucléotides de la SÉQ ID N° 1 pour une utilisation dans le traitement de troubles cancéreux, génétiques ou neurologiques, par un procédé d'édition génomique pour introduire le dit acide nucléique dans des cellules hôtes et augmenter l'expression d'une protéine d'intérêt, dans lequel l'acide nucléique est introduit en aval du gène de la région codante d'intérêt.

12. Procédé in vitro consistant à réduire l'expression, pratiquement supprimer l'expression ou pratiquement arrêter l'expression d'une protéine d'intérêt dans une cellule hôte, comprenant l'étape consistant à : supprimer ou interférer avec l'acide nucléique avec la séquence des bases à 28 oligonucléotides de la SÉQ ID N° 1.
